# EUROPÄISCHE PATENTSCHRIFT

(11) **EP 1 119 640 B1**
(45) Veröffentlichungstag und Bekanntmachung des Hinweises auf die Patenterteilung: **29.11.2006**
(21) Anmeldenummer: 99948931.3
(22) Anmeldetag: 05.10.1999
(51) Int. Cl.: C12Q 1/42

(54) **VERFAHREN ZUR BESTIMMUNG VON ALKALISCHER PHOSPHATASE**
METHOD FOR DETERMINING ALKALINE PHOSPHATASE
PROCEDE POUR DETECTER LA PHOSPHATASE ALCALINE

(30) Priorität: 08.10.1998 DE 19846300
(43) Veröffentlichungstag der Anmeldung: 01.08.2001
(73) Patentinhaber: Roche Diagnostics GmbH, 68298 Mannheim (DE)
(72) Erfinder: WEISHEIT, Ralph, D-82380 Peissenberg (DE); TREIBER, Wolfgang, D-82362 Weilheim (DE)
(86) Internationale Anmeldenummer: PCT/EP1999/007366
(87) Internationale Veröffentlichungsnummer: WO 2000/022161

(56) Entgegenhaltungen:
- EP-A- 0 695 805
- WO-A-97/45728
- WO-A-98/02570
- US-A- 5 766 872
- DATABASE CHEMABS [Online] CHEMICAL ABSTRACTS SERVICE, COLUMBUS, OHIO, US GLICK, MELVIN R. ET AL: "Analytical systems ranked by freedom from interferences" retrieved from STN Database accession no. 107:112184 XP002128041 & CLIN. CHEM. (WINSTON-SALEM, N. C.) (1987), 33(8), 1453-8 ,
- JAY D.W. ET AL: 'Characterization and mathematical correction of hemolysis interference in selected Hitachi 717 assays' CLINICAL CHEMISTRY Bd. 39, Nr. 9, September 1993, USA, Seiten 1804 - 1810

## Beschreibung

Die vorliegende Erfindung betrifft ein Verfahren zur Bestimmung von alkalischer Phosphatase in einer Probe durch optische Messung des sich bildenden p-Nitrophenols, das dadurch gekennzeichnet ist, daß man zur Beseitigung von Hämoglobin-Störungen eine Hauptmeßwellenlänge von 450 ± 10 nm in Kombination mit dem Rate-Blank-Verfahren verwendet, eine Methode zur Beseitigung von Störungen durch freies Hämoglobin oder Blutersatzmittel, sowie die Verwendung der Kombination einer Hauptmeßwellenlänge mit dem Rate-Blank-Verfahren zur Beseitigung von Störungen durch freies Hämoglobin oder Blutersatzmittel.

Es ist bekannt, daß durch Hämolyse diagnostische Verfahren zur Bestimmung von Analyten teilweise erheblich gestört werden. Unter Hämolyse wird jegliche Zerstörung von Erythrocyten beispielsweise durch mechanische, osmotische, chemische oder enzymatische Einwirkung auf die Zellmembran der Erythrocyten verstanden. Infolge der Hämolyse tritt der Blutfarbstoff Hämoglobin (Hb) aus und ist aus einer Probe nicht mehr zu entfernen. Problematisch ist die Anwesenheit von Hämoglobin zum einen aufgrund des mit den Spektren der nachzuweisenden Substanzen und Indikatoren (Chromogenen) zum Teil erheblich überlappenden Absorptionsspektrums von Hämoglobin, wodurch es zu Fehlmessungen in photometrischen Tests kommen kann. Zum anderen kann Hämoglobin auch mit Probenbestandteilen chemisch reagieren, wodurch ebenfalls Substanzen entstehen, die Fehlmessungen erzeugen können.

In letzter Zeit sind für Therapiezwecke beispielsweise nach hohem Blutverlust immer häufiger Blutersatzmittel im Einsatz, die auf Basis von Hämoglobin hergestellt werden. Das Hämoglobin in Blutersatzmitteln kann nativer, oder synthetischer Natur sein. Oftmals werden auch Hb-analoge Verbindungen eingesetzt. Im Gegensatz zur Hämolyse, bei der im allgemeinen ein Hämoglobingehalt von bis zu 500 mg/dl auftritt, ist bei einer Therapie mit Blutersatzmitteln mit einem Hb-Gehalt im Blutserum bzw. -plasma von mehr als 2000 mg/dl zu rechnen. Störungen in Proben, die Blutersatzmittel enthalten, sind daher oftmals wesentlich ausgeprägter als in hämolytischen Proben, da das Hämoglobin oder das synthetische Analogon von vornherein in freier Form vorliegt.

Besonders gravierend ist der Störeinfluß von freiem Hämoglobin bei der photometrischen Bestimmung von alkalischer Phosphatase. Bei der Bestimmung der alkalischen Phosphatase wird die Bildung von 4-Nitrophenol bei 405 bis 415 nm gemessen (Extinktionszunahme). Hämoglobin absorbiert ebenfalls bei 415 nm. In Gegenwart von Hämoglobin wird die Bestimmung der alkalischen Phosphatase in zweierlei Hinsicht gestört: Zum einen ändert sich im alkalischen Milieu das Hb-Spektrum zeitabhängig (Extinktionsabnahme), zum anderen wird ab einem bestimmten Hb-Gehalt die Photometergrenze des Meßgerätes erreicht.

Zur Beseitigung spektraler und chemischer Einflüsse von Hämoglobin auf die Analyse von Serum- oder Plasmaproben sind im Stand der Technik unterschiedliche Verfahren publiziert.

Jay und Provasek beschreiben in Clin Chem 39/9, 1804-1810 (1993), daß die Hämoglobin-störung bei der Bestimmung von alkalischer Phosphatase durch eine zeitabhängige Änderung des Hb-Spektrums verursacht wird. Diese Störung kann durch mathematische Korrekturatgorithmen (Bestimmung der Hb-Konzentration in der Probe und Korrektur des gemessenen Wertes für alkalische Phosphatase um einen bestimmten Betrag, der der gemessenen Hb-Menge äquivalent ist) beseitigt werden.

Die von Jay und Provasek erwähnte mathematische Korrektur beseitigt zwar den Hb-Einfluß bis mindestens 800 mg/dl Hb, ist jedoch wenig anwenderfreundlich, da sie eine zusätzliche Messung des Hb-Gehaltes voraussetzt und anschließend noch einen mathematischen Korrekturschritt erfordert.

Jay und Provasek (supra) beschreiben eine weitere Methode zur Entstörung durch sogenannte Rate-Blank-Messung. Die Korrektur von Hämolyse-Störungen durch Rate-Blank-Messungen ist auch in der EP-A-0 695 805 beschrieben. Hier wird vor der eigentlichen photometrischen Bestimmung einer in der Probe enthaltenen Komponente die Probe einer Vorreaktion unterworfen, durch die der Hämolysegrad der Probe bestimmt wird. Der nachfolgend erhaltene Meßwert wird dann korrigiert um einen Wert, der durch Korrelation des Hämolysegrades mit dem Meßfehlerbeitrag von störenden Komponenten ermittelt wurde.

Durch Rate-Blank-Messungen kann die Hb-Störung beseitigt werden, allerdings nur bis zu einem Hb-Gehalt von ca. 1200 mg/dl, da bei höherem Hb-Gehalt die Photometergrenze erreicht wird. Dies kann zwar ausreichend für die Beseitigung von Hämolysestörungen sein, ist jedoch keinesfalls ausreichend für die Beseitigung von Blutersatzmittel-Störungen.

Eine weitere Möglichkeit zur Beseitigung von Hämoglobinstörungen wurde für die Bestimmung von Albumin publiziert (PCT-Anmeldung WO 97/45728), wo durch spezielle Kombinationen von Haupt- und Nebenwellenlängen eine Beseitigung von Hämoglobinstörungen erzielt werden konnte. Die hier genannten Wellenlängenkombinationen können jedoch nicht für die Bestimmung der alkalischen Phosphatase verwendet werden, da bei diesen Wellenlängen kein Meßsignal mehr für 4-Nitrophenol erhalten wird.

Die Offenlegungsschrift WO 97/45733 beschreibt, daß mit den Nebenwellenlängen 546 und 570 nm bei einzelnen UV-Tests die Störungen durch Hämoglobin beseitigt werden können. Dieses Verfahren ist jedoch nur auf enzymatische UV-Tests mit einer Hauptmeßwellenlänge von 340 nm anwendbar. Während hierbei eine vollständige Hb-Entstörung allein durch die Nebenwellenlängen 546 bzw. 570 nm erreicht werden kann, ist dies im Fall von enzymatischen Farbtests wie beispielsweise zur Bestimmung von alkalischer Phosphatase, bei denen die Hauptmeßwellenlänge im Bereich von 415 nm liegt, nicht möglich.

Im US-Patent 5,766,872 wird erwähnt, daß bei der Amylase-Bestimmung die Nebenwellenlänge von 577 nm zu einer Verringerung der Hämolysestörung führt. Aus den angeführten Meßdaten ist jedoch zu erkennen, daß bereits bei einem Hb-Gehalt von 500 mg/dl eine signifikante Meßwertabweichung von bis zu 8 % vorliegt. Das reicht zwar für die Hämolyse-Entstörung, doch ist aufgrund der verwendeten Hauptmeßwellenlänge von ca. 415 nm zu erwarten, daß bei höherem Hb-Gehalt (wie er bei einer Therapie mit Blutersatzmitteln auftritt) diese Meßwertabweichung ebenfalls größer wird und eine ausreichende Hb-Entstörung dann nicht mehr gegeben ist.

Im Stand der Technik ist kein Verfahren zur Bestimmung von alkalischer Phosphatase bekannt, das auch in Anwesenheit von hohen Hb-Konzentrationen, wie sie beispielsweise in Blutersatzmittel enthaltenden Proben vorkommen, störungsfrei durchgeführt werden kann.

Aufgabe war es daher, ein verbessertes Verfahren zur Bestimmung der alkalischen Phosphatase in einer Probe durch optische Messung von p-Nitrophenol zu entwickeln, das die Nachteile des Standes der Technik weitgehend überwindet. Insbesondere sollte ein einfaches und anwenderfreundliches Verfahren zur Beseitigung von Störungen durch Hämoglobin und auf Hämoglobin basierenden Blutersatzmitteln bei der Bestimmung der alkalischen Phosphatase bereit gestellt werden.

Gelöst wird die Aufgabe durch das in den Ansprüchen näher definierte Verfahren zur Bestimmung von alkalischer Phosphatase in einer Probe durch optische Messung von p-Nitrophenol. Das erfahren ist dadurch gekennzeichnet, daß man eine Hauptmeßwellenlänge von 450 ± 10 nm in Kombination mit dem Rate-Blank-Verfahren verwendet.

Es hat sich überraschenderweise gezeigt, daß eine effektive Hb-Entstörung für die Bestimmung der alkalischen Phosphatase dann möglich ist, wenn zum einen die Hauptwellenlänge geändert wird und zum anderen das Rate-Blank-Verfahren angewendet wird. Für eine zufriedenstellende Hb-Entstörung ist es nicht ausreichend, nur die Hauptwellenlänge zu ändern oder nur das Rate-Blank-Verfahren anzuwenden.

Aufgrund des Absorptionsspektrums von 4-Nitrophenol kann die alkalische Phosphatase nicht nur bei 415 nm gemessen werden, sondern auch bei 450 ± 10 nm. Zwar befindet sich damit die Hauptmeßwellenlänge nicht im üblicherweise verwendeten Absorptionsmaximum der Nachweisreaktion, sondern an dessen Flanke, aber das erhaltene Meßsignal ist dennoch ausreichend für die exakte Bestimmung der alkalischen Phosphatase.

Bereits durch Wahl der neuen Hauptmeßwellenlänge 450 ± 10 nm kommt es zu einer leichten Verringerung der Hämoglobin-Störung, aber eine vollständige Entstörung erhält man überraschenderweise erst durch die Kombination der Hauptwellenlänge 450 ± 10 nm mit dem Rate-Blank-Verfahren.

Mit Hilfe des erfindungsgemäßen Verfahrens ist es erstmals auf einfache Weise möglich, die durch Hämoglobin bzw. Hb-analoge Verbindungen verursachten Störungen bei der Bestimmung von alkalischer Phosphatase durch optische Messung von p-Nitrophenol bis zu einem Hb-Gehalt von mind. 3000 mg/dl zu beseitigen. Die Obergrenze zur Beseitigung der Hb-Störungen liegt bei der durch die Leistungsfähigkeit des verwendeten Photometers bestimmten Grenze. Bis zu 6500 mg/dl Hämoglobin-Gehalt ist daher eine gute Entstörung durch das erfindungsgemäße Verfahren denkbar.

Im Sinne der Erfindung wird beim Rate-Blank-Verfahren vor der eigentlichen photometrischen Bestimmung einer in der Probe enthaltenen Komponente die Probe einer Vorreaktion unterworfen, durch die der Hämolysegrad der Probe bestimmt wird. Der nachfolgend erhaltene Meßwert der zu bestimmenden Komponente wird dann um einen Wert korrigiert, der durch Korrelation des Hämolysegrades mit dem Meßfehlerbeitrag von störenden Komponenten ermittelt wurde. Das Rate-Blank-Verfahren an sich zur Korrektur von Hämolyse-Störungen ist beispielsweise in der EP-A-0 695 805 und bei Jay und Provasek in Clin Chem 39/9, 1804-1810 (1993), beschrieben.

Die für das Rate-Blank-Verfahren verwendete Nebenmeßwellenlänge ist für die Erfindung unerheblich. Auch die Länge des zeitlichen Meßfensters für die Vorreaktion und die Hauptreaktion ist nicht entscheidend. Als geeignet hat es sich erwiesen, die Extinktionsänderungen der Vor- und Hauptreaktion über einen Zeitraum von 1 bis 4 Minuten zu messen.

Das erfindungsgemäße Verfahren eignet sich zur Bestimmung beliebiger Proben, in denen freies Hämoglobin vorliegt. Der Begriff "freies Hämoglobin" im Sinne der Erfindung wird in Abgrenzung zu solchem Hämoglobin verwendet, das in intakten Erythrocyten enthalten ist. Beispiele für Proben, die freies Hämoglobin enthalten, sind hämolytische Serum- oder Plasmaproben oder Proben, die Blutersatzmittel enthalten. Beispiele für Blutersatzmittel, die im Sinne der vorliegenden Erfindung unter den Begriff "freies Hämoglobin" fallen, sind derivatisierte, polymerisierte, modifizierte oder quervernetzte Derivate von Hämoglobinen, insbesondere von Humanhämoglobin oder Rinderhämoglobin, z. B. DCL-Hämoglobin (Diaspirin-crosslinked Hämoglobin), sowie rekombinant hergestelltes Hämoglobin.

Ebenfalls ein Gegenstand der Erfindung ist eine Methode zur Beseitigung von Störungen, die durch freies Hämoglobin hervorgerufen werden, in einem Verfahren zur Bestimmung von alkalischer Phosphatase durch optische Messung von p-Nitrophenol. Die Methode ist dadurch gekennzeichnet, daß man eine Hauptmeßwellenlänge von 450 ± 10 nm in Kombination mit dem Rate-Blank-Verfahren verwendet.

Ein weiterer Gegenstand der Erfindung ist die Verwendung einer Hauptmeßwellenlänge von 450 ± 10 nm in Kombination mit dem Rate-Blank-Verfahren zur Beseitigung von Störungen durch freies Hämoglobin oder durch auf Hämoglobinbasis hergestellte Blutersatzmittel in einem Verfahren zur Bestimmung von alkalischer Phosphatase durch optische Messung von p-Nitrophenol.

Das folgende Beispiel erläutert die Erfindung weiter.

### Beispiel

### a) Herstellung der Hämoglobin haltigen Proben

Ein Teil eines Serumpools wurde mit einer Hb-haltigen Lösung derart versetzt, daß ein Hb-Gehalt von mindestens 3000 mg/dl erreicht wurde. Ein anderer gleichgroßer Teil desselben Serumpools wurde mit der äquivalenten Menge NaCl-Lösung (154 mmol/l) versetzt. Beide Teile wurden anschließend in unterschiedlichem Verhältnis derart miteinander vermischt, daß eine Hb-Konzentrationsreihe aus 11 Proben erhalten wird, deren niedrigste Probe kein Hb und deren höchste Probe mindestens 3000 mg/dl Hb enthält.

### b) Bestimmung der alkalischen Phosphatase nach der SFBC-Methode

Bestimmung nach Empfehlungen der Société Française de Biologie Clinique gemäß Ann. Biol. Clin. Vol 35, 271 (1977)

Die Bestimmung der alkalischen Phosphatase wurde an einem Boehringer Mannheim/Hitachi 911-Analysengerät durchgeführt.

Es wurden folgende Reagenzien verwendet:
- Reagenz 1:: 930 mmol/l 2-Amino-2-methyl-1-propanol-Puffer, pH 10,5;
1,03 mmol/l Magnesiumaspartat
- Reagenz 2:: 930 mmol/l 2-Amino-2-methyl-1-propanol-Putier, pH 10,5;
1,03 mmol/l Magnesiumaspartat; 98 mmol/l 4-Nitrophenylphosphat

Die Testdurchführung war wie folgt: Zu 11 µl Probe wurden 250 µl Reagenz 1 und nach 5 min 50 µl Reagenz 2 gegeben. Die Bestimmung des Analyten erfolgte für die Vergleichsmessungen nach einer Dauer von weiteren 50 sec, wobei die Extinktionsänderung während der nachfolgenden 4 min gemessen wurde. Zur Messung wurden Kombinationen folgender Hauptmeßwellenlängen (λ₁) und Nebenmeßwellenlängen (λ₂) verwendet: λ₁/λ₂ = 415/660 nm (bisherige Geräteeinstellung), 415/570 nm und 450/660 nm (Vergleich). Als weiterer Vergleich wurde die Bestimmung der alkalischen Phosphatase nach der von Jay und Provasek erwähnten Rate-Blank-Messung durchgeführt (als "415/660 nm RB" bezeichnet).

Bei der Erfindung wurde für die Analytbestimmung die Meßwellenlängenkombination λ₁/λ₂ = 450/660 nm verwendet. Für die Rate-Blank-Messung wurde die Extinktionsänderung der Vorreaktion in der Zeit von 3,0 - 4,9 min nach Zugabe von Reagenz 1 zur Probe und die Extinktionsänderung der Hauptreaktion in der Zeit von 7,9 - 9,8 min nach Zugabe von Reagenz 1 zur Probe gemessen. Dies entspricht am Boehringer Mannheim/Hitachi 911 1 Analysengerät den Meßpunkten [10] - [16] und [25] - [31]. Das Ergebnis ist in der als "450/660 nm RB" bezeichneten Spalte aufgeführt.

Die Ergebnisse der erfindungsgemäßen Messung sowie der Vergleichsmessungen sind in der nachfolgenden Tabelle 1 gezeigt. Es ist zu erkennen, daß bei Verwendung der erfindungsgemäßen Kombination der neuen Hauptmeßwellenlänge von 450 nm mit dem Rate-Blank-Verfahren die Störung durch Hb-haltige Blutersatzmittel im Vergleich zu den anderen Meßwellenlängenkombinationen bzw. zur Rate-Blank-Messung bei 415 nm Hauptwellenlänge drastisch reduziert wird.

**Tabelle 1**

| Gemessener Gehalt an Alkalischer Phosphatase bei 37°C in U/l | | | | | |
|---|---|---|---|---|---|
| Hb-Gehalt* [rng/dl] | 415/660 nm | 415/570 nm | 450/660 nm | 415/660 nm RB | 450/660 nm RB |
| 0 | 42 | 42 | 42 | 42 | 42 |
| 300 | 32 | 32 | 35 | 44 | 43 |
| 600 | 22 | 24 | 29 | 46 | 44 |
| 900 | 14 | 16 | 24 | 46 | 46 |
| 1200 | 8 | 11 | 22 | 44 | 45 |
| 1500 | 3 | 7 | 19 | 5 | 47 |
| 1800 | -2 | 2 | 17 | 0 | 47 |
| 2100 | -2 | 3 | 17 | 0 | 48 |
| 2400 | -2 | 3 | 15 | 1 | 50 |
| 2700 | -1 | 3 | 16 | 0 | 49 |
| 3000 | -2 | 3 | 19 | 1 | 51 |

| | | | | | |
|---|---|---|---|---|---|
| * hier wurde ein vernetztes Hämoglobin eingesetzt | | | | | |

## Patentansprüche

1. Verfahren zur Bestimmung von alkalischer Phosphatase in einer Probe durch optische Messung von p-Nitrophenol **dadurch gekennzeichnet, daß** man eine Hauptmeßwellenlänge von 450 ± 10 nm in Kombination mit dem Rate-Blank-Verfahren verwendet.

2. Verfahren nach Anspruch 1, **dadurch gekennzeichnet, daß** die Bestimmung in einer Serum- oder Plasmaprobe durchgeführt wird.

3. Verfahren nach einem der vorhergehenden Ansprüche **dadurch gekennzeichnet, daß** man eine Probe bestimmt, die freies Hämoglobin oder ein auf Hämoglobinbasis hergestelltes Blutersatzmittel enthält.

4. Verfahren nach einem der vorhergehenden Ansprüche, **dadurch gekennzeichnet, daß** das Blutersatzmittel ein derivatisiertes, modifiziertes oder quervemetztes Humanhämoglobin oder Rinderhämoglobin oder rekombinant hergestelltes Hämoglobin ist.

5. Verfahren nach einem der vorhergehenden Ansprüche, **dadurch gekennzeichnet, daß** die Probe einen Hämoglobingehalt bis zu 6500 mg/dl aufweist.

6. Methode zur Beseitigung von Störungen, die durch freies Hämoglobin oder Blutersatzmittel hervorgerufen werden, in einem Verfahren zur Bestimmung von alkalischer Phosphatase gemäß Anspruch 1 **dadurch gekennzeichnet, daß** man eine Hauptmeßwellenlänge von 450 ± 10 nm in Kombination mit dem Rate-Blank-Verfahren verwendet.

7. Verwendung einer Hauptmeßwellenlänge von 450 ± 10 nm in Kombination mit dem Rate-Blank-Verfahren zur Beseitigung von Störungen durch freies Hämoglobin oder Blutersatzmittel in einem Verfahren gemäß Anspruch 1 zur Bestimmung von alkalischer Phosphatase.

## Claims

1. Procedure for the determination of alkaline phosphatase in a sample by optical measurement wherein a main measuring wave length of 450 ± 10 nm is used in combination with the Rate-Blank procedure.

2. Procedure as claimed in claim 1 wherein the determination is performed in a serum or plasma sample.

3. Procedure as claimed in one of the aforementioned claims wherein a sample containing free hemoglobin or a blood substitute produced on the basis of hemoglobin is analyzed.

4. Procedure as claimed in one of the aforementioned claims wherein the blood substitute contains a derivatized, modified or cross-linked human hemoglobin, bovine hemoglobin or a recombinantly produced hemoglobin.

5. Procedure as claimed in one of the aforementioned claims wherein the sample has a hemoglobin concentration of up to 6500 mg/dl.

6. Method for the elimination of interferences caused by free hemoglobin or blood substitutes, in a procedure for the determination of alkaline phosphatase wherein a main measuring wave length of 450 ± 10 nm is used in combination with the Rate-Blank procedure.

7. Use of a main measuring wave length of 450 ± 10 nm combined with the Rate-Blank procedure for the elimination of interferences caused by free hemoglobin or blood substitutes in a procedure for the determination of alkaline phosphatase.

## Revendications

1. Procédé pour la détermination de phosphatase alcaline dans un échantillon par mesure optique du p-nitrophénol, **caractérisé en ce qu'**on utilise une longueur d'onde de mesure principale de 450 ± 10 nm en combinaison avec le procédé « Rate-Blank ».

2. Procédé selon la revendication 1, **caractérisé en ce qu'**on procède à la détermination dans un échantillon de sérum ou de plasma.

3. Procédé selon l'une quelconque des revendications précédentes, **caractérisé en ce qu'**on détermine un échantillon qui contient de l'hémoglobine libre ou bien un succédané du sang préparé à base d'hémoglobine.

4. Procédé selon l'une quelconque des revendications précédentes, **caractérisé en ce que** le succédané du sang est une hémoglobine humaine dérivée, modifiée ou réticulée, ou bien une hémoglobine bovine ou encore une hémoglobine préparée par voie recombinante.

5. Procédé selon l'une quelconque des revendications précédentes, **caractérisé en ce que** l'échantillon présente une teneur en hémoglobine s'élevant jusqu'à 6500 mg/dl.

6. Procédé pour supprimer des parasites qui sont dus à la présence d'hémoglobine libre ou d'un succédané du sang, dans un procédé pour la détermination de la teneur en phosphatase alcaline selon la revendication 1, **caractérisé en ce qu'**on utilise une longueur d'onde de mesure principale de 450 ± 10 nm en combinaison avec le procédé « Rate-Blank ».

7. Utilisation d'une longueur d'onde de mesure principale de 450 ± 10 nm en combinaison avec le procédé « Rate-Blank » pour supprimer des parasites qui sont dus à la présence d'hémoglobine libre ou d'un succédané du sang, dans un procédé selon la revendication 1 pour la détermination de phosphatase alcaline.
